# FASCICULE DE BREVET EUROPEEN

(11) **EP 3 077 333 B1**
(45) Date de publication et mention de la délivrance du brevet: **20.02.2019**
(21) Numéro de dépôt: 14805938.9
(22) Date de dépôt: 03.12.2014
(51) Int. Cl.: C01B 21/093, H01M 10/0568, H01M 10/0567

(54) **PROCÉDÉ DE PRÉPARATION D'UN COMPOSÉ FLUORÉ ET SOUFRÉ ET DE SES SELS EN MILIEU AQUEUX**
VERFAHREN ZUR HERSTELLUNG EINER FLUOR- UND SCHWEFELTRAGENDEN VERBINDUNG UND SALZE DAVON IN EINEM WÄSSRIGEN MEDIUM
METHOD FOR PRODUCING A FLUORINE- AND SULPHUR-BEARING COMPOUND AND SALTS THEREOF IN AN AQUEOUS MEDIUM

(30) Priorité: 05.12.2013 FR 1362136
(43) Date de publication de la demande: 12.10.2016
(73) Titulaire: Rhodia Operations, 75009 Paris (FR)
(72) Inventeur: BUISINE, Olivier, F-69230 Saint Genis Laval (FR); METZ, François, F-69540 Irigny (FR)
(74) Mandataire: Menville, Laure
(86) Numéro de dépôt international: PCT/EP2014/076355
(87) Numéro de publication internationale: WO 2015/082519

(56) Documents cités:
- EP-A1- 2 660 196
- MARTIN BERAN ET AL: "A New Method of the Preparation of Imido-bis(sulfuric acid) Dihalogenide, (F, Cl), and the Potassium Salt of Imido-bis(sulfuric acid) Difluoride", ZEITSCHRIFT F&#XFFFD;R ANORGANISCHE UND ALLGEMEINE CHEMIE, vol. 631, no. 1, 1 janvier 2005 (2005-01-01), pages 55-59, XP055014688, ISSN: 0044-2313, DOI: 10.1002/zaac.200400325
- HONG-BO HAN ET AL: "Lithium bis(fluorosulfonyl)imide (LiFSI) as conducting salt for nonaqueous liquid electrolytes for lithium-ion batteries: Physicochemical and electrochemical properties", JOURNAL OF POWER SOURCES, ELSEVIER SA, CH, vol. 196, no. 7, 10 décembre 2010 (2010-12-10), pages 3623-3632, XP028129764, ISSN: 0378-7753, DOI: 10.1016/J.JPOWSOUR.2010.12.040 [extrait le 2010-12-21]
- WILLIAM DAVIES ET AL: "CCLXXXVI.-Aromatic sulphonyl fluorides. A convenient method of preparation", JOURNAL OF THE CHEMICAL SOCIETY (RESUMED), no. 0, 1 janvier 1931 (1931-01-01), pages 2104-2109, XP055134672, DOI: 10.1039/JR9310002104

## Description

La présente invention se rapporte au domaine de la préparation de composés fluorés et soufrés en milieu aqueux et de la préparation des sels desdits composés lesquels sont utiles dans des applications telles que l'électronique et l'électrochromisme. De manière plus particulière, la présente invention a pour objet la préparation de sels de l'acide bis-fluorosulfonylimide (FSIH), notamment du bis-fluorosulfonylimidure de lithium (LiFSI).

La production de FISH et de LiFSI est largement décrite dans la littérature. Parmi les différentes technologies décrites, la majorité met en oeuvre une réaction de fluoration soit par HF soit par des fluorures métalliques. L'utilisation de fluorures métalliques est problématique car elle est souvent peu efficace et met en oeuvre des réactifs onéreux tel que l'acide fluorosulfurique. Par exemple, la fluoration par le fluorure de potassium dans le nitrométhane ou autres solvants organiques polaires est peu efficace en termes de rendement (WO 2002/053494). D'autres technologies ont été développées, par exemple en mettant en oeuvre le chlorosulfonyle isocyanate en présence d'oléum et du fluorure d'ammonium (JP 2012-162470) ou encore en utilisant l'urée et l'acide fluorosulfonique, mais ces technologies pâtissent de la forte corrosion du milieu ainsi que de l'exothermie de la réaction. Ces inconvénients rendent ces technologies peu adaptées pour une production industrielle de l'acide bis-fluorosulfonylimide et de ses sels.

WILLIAM DAVIES ET AL: "CCLXXXVI.-Aromatic sulphonyl fluorides. A convenient method of preparation", JOURNAL OF THE CHEMICAL SOCIETY (RESUMED), no. 0, (1931) pages 2104-2109, divulgue la préparation du benzène monosulfonyl fluorure par réaction en présence d'eau du chlorure correspondant avec un fluorure inorganique. Aussi le besoin de produire l'acide bis-fluorosulfonylimide (FSIH) et de manière plus générale des composés fluorés et soufrés et leurs sels selon un procédé alternatif remédiant aux inconvénients cités ci-dessus est grand. De manière surprenante, la Demanderesse a mis au point un nouveau procédé de production de composés fluorés et soufrés et de leurs sels opérant dans des conditions douces et sans exothermie. Le procédé selon la présente invention présente l'avantage de pouvoir être mis facilement en oeuvre à l'échelle industrielle. Il met en oeuvre une réaction de fluoration qui opère en milieu aqueux. L'utilisation d'eau comme solvant permet non seulement d'améliorer la performance de la réaction de fluoration en comparaison avec celles obtenues par la mise en oeuvre d'une réaction de fluoration opérée en milieu anhydre mais également de séparer aisément le composé fluoré et soufré formé, généralement sous forme salifiée, du milieu de fluoration.

La présente invention a pour objet un procédé de préparation selon la revendication 1 d'un composé fluoré et soufré de formule F-SO-R (I) ou F-SO₂-R (II) comprenant la réaction, en présence d'eau, d'au moins un sel apportant un anion fluorure et d'au moins un composé halogénosulfoxyde de formule X-SO-R' (I0) respectivement d'un composé halogénosulfonyle de formule X-SO₂-R' (II0), où X est un atome d'halogène différent du fluor et R et R' sont chacun un groupement lié par une liaison covalente à l'atome de soufre, ladite liaison liant ledit atome de soufre avec un atome d'azote.
Ledit composé halogénosulfoxyde de formule (I0) ou ledit composé halogénosulfonyle de formule (II0) employé comme réactif pour la mise en oeuvre du procédé selon l'invention peut se présenter sous forme acide ou sous forme salifiée, par exemple sous forme de sel des métaux alcalins, de sel de métaux alcalino-terreux ou de sel organique sous forme de liquide ionique, en particulier de sel d'onium et plus particulièrement de sel d'ammonium, de phosphonium, d'imidazolium, de pyridinium, de guanidinium.
Le procédé de l'invention opère une réaction de fluoration ou réaction d'échange halogène/fluor, le(s) atome(s) d'halogène différent du fluor étant échangé(s) par le fluor. On donne ci-après le schéma réactionnel du procédé de l'invention pour faciliter la compréhension sans pour autant lier la portée de l'invention à celui-ci.

X-SO-R' + MF → F-SO-R + MX ou X-SO₂-R' + MF → F-SO₂-R + MX

Dans les équations ci-dessus, M est le cation associé à l'anion fluorure. Les groupements R et R' sont identiques lorsque R' ne comporte aucun atome d'halogène autre que le fluor susceptible d'être touché par ladite réaction de fluoration selon le procédé de l'invention. R et R' sont différents et diffèrent l'un de l'autre par la nature de l'halogène lorsque R' comporte un ou plusieurs atomes d'halogène, autre que le fluor, touchés par ladite réaction de fluoration.
De manière plus préférée, le procédé de l'invention a pour objet la préparation dudit composé fluoré et soufré de formule F-SO₂-R (II) à partir dudit composé halogénosulfonyle de formule X-SO₂-R' (II0).

Dans le procédé selon l'invention, R et R' sont chacun un groupement lié par une liaison covalente à l'atome de soufre, ladite liaison liant ledit atome de soufre avec un atome d'azote. Selon un mode de réalisation préféré, ledit atome d'azote est lié à un autre groupe -SO₂- ou -SO-. Ainsi, R et R' peuvent être choisi tels que les composés (I) et (II) comprennent un atome d'azote lié à deux groupes - SO₂- ou -SO-.

Le groupement R est choisi dans le groupe constitué par :
- un groupement -NHSO₂F,
- un groupement -NHSOF,
- un groupement -NM'SO₂F,
- un groupement -NM'SOF,
- un groupement -NR₁R₁₀,
- un groupement -NHSO₂R₁,
- un groupement -NHSOR₁, et
- un groupement -NHCOR₁ ;
où M' est un métal alcalin ou alcalino-terreux ou un ion onium, et où R₁ et R₁₀, identiques ou différents, sont choisis dans le groupe constitué par un atome d'hydrogène, une chaîne hydrocarbonée linéaire, ramifiée ou cyclique, saturée ou insaturée ayant de 1 à 15 atomes de carbone, une chaîne fluoroalkyle, perfluoroalkyle ou fluoroalkényle ayant de 1 à 15 atomes de carbone et un groupement aromatique.

Selon un mode préféré de réalisation du procédé de l'invention, le groupement R est choisi dans le groupe constitué par un groupement -NHSO₂F, un groupement -NM'SO₂F (M' étant un métal alcalin ou alcalino-terreux), un groupement-NR₁R₁₀, un groupement -NHSO₂R₁, et un groupement -NHCOR₁ ; où R₁ et R₁₀, identiques ou différents, sont choisis dans le groupe constitué par un atome d'hydrogène, une chaîne hydrocarbonée linéaire, ramifiée ou cyclique, saturée ou insaturée ayant de 1 à 15 atomes de carbone, une chaîne fluoroalkyle, perfluoroalkyle ou fluoroalkényle ayant de 1 à 15 atomes de carbone et un groupement aromatique.

Le groupement aromatique couvre notamment un radical aryle ou hétéroaryle monocyclique ou polycyclique, le radical aryle étant de préférence un cycle aromatique de 5 à 6 chaînons. Ledit radical aryle ou hétéroaryle peut lui-même porter un ou plusieurs substituant(s), par exemple une chaîne carbonée, linéaire ou ramifiée, saturée ou insaturée, un groupe halogène, hydroxy, trifluorométhyle, trifluorométhoxy, méthoxy, carboxy, aminé, oxo, nitro ou cyano.

De manière très avantageuse, le groupement R est un groupement -NHSO₂F, un groupement -NM'SO₂F (M' étant un métal alcalin ou alcalino-terreux) ou un groupement -NHSO₂R₁ où R₁ est une chaîne fluoroalkyle ou perfluoroalkyle, préférentiellement une chaîne perfluoroalkyle ayant de 1 à 5 atomes de carbone, très préférentiellement R₁ est le groupement -CF₃.
Selon ce mode de réalisation, le groupement R' est choisi dans le groupe constitué par un groupement -NHSO₂X (X ayant la définition donnée ci-dessus), un groupement -NR'₁R'₁₀, un groupement -NHSO₂R'₁, et un groupement-NHCOR'₁ ; où R'₁ et R'₁₀, identiques ou différents, sont choisis dans le groupe constitué par un atome d'hydrogène, une chaîne hydrocarbonée linéaire, ramifiée ou cyclique, saturée ou insaturée ayant de 1 à 15 atomes de carbone, une chaîne halogénoalkyle, perhalogénoalkyle ou halogénoalkényle dans laquelle le(s) atome(s) d'halogène est(sont) choisi(s) parmi le brome, le chlore, le fluor et l'iode et ayant de 1 à 15 atomes de carbone et un groupement aromatique. De manière très avantageuse, le groupement R' est un groupement -NHSO₂X (X ayant la définition donnée ci-dessus, préférentiellement X est le chlore) ou un groupement -NHSO₂R'₁ où R'₁ est une chaîne halogénoalkyle ou perhalogénoalkyle.

Ledit mode est particulièrement avantageux lorsque la réaction de fluoration est mise en oeuvre en présence dudit composé halogénosulfonyle de formule X-SO₂-R' (II0) pour préparer ledit composé fluoré et soufré de formule F-SO₂-R (II).
De manière très préférée, on met en oeuvre le procédé de l'invention en utilisant un composé halogénosulfonyle de formule (II0) X-SO₂-NH-SO₂-X (X ayant la définition donnée ci-dessus, préférentiellement X est le chlore) de manière à préparer le composé F-SO₂-NH-SO₂-F (II), appelé acide bis-fluorosulfonylimide (FSIH) ou un sel dudit composé (F-SO₂-NM'-SO₂-F où M' est un métal alcalin ou alcalino-terreux). On peut encore employer un sel métallique ou organique dudit composé halogénosulfonyle de formule (II0). De manière également très préférée, on met en oeuvre le procédé de l'invention en utilisant un composé halogénosulfonyle de formule (II0) X-SO₂-NH-SO₂-CF₃ (X ayant la définition donnée ci-dessus, préférentiellement X est le chlore) de manière à préparer le composé F-SO₂-NH-SO₂-CF₃ (II).

Toutefois, la réactivité des composés halogénosulfoxydes et halogénosulfonyles peut varier en fonction de la richesse en électron du groupe R'. Ainsi, l'hydrolyse des composés dans lesquels R' est un groupe aromatique est relativement lente. Au contraire, les réactifs dans lesquels le groupe X-SO- ou X-SO₂- est lié à un atome d'azote, notamment les composés dans lesquels ledit atome d'azote est lié à un autre groupe -SO₂- ou -SO-, sont connus pour réagir violemment avec l'eau.

Conformément au procédé selon l'invention, la nature dudit sel apportant au moins ledit anion fluorure peut être de nature variée. De manière avantageuse, ledit sel est choisi parmi les fluorures métalliques, les fluorures d'onium et leurs mélanges.

Lesdits fluorures métalliques, avantageusement employés comme sels apportant des anions fluorures dans le procédé selon l'invention, sont préférentiellement des fluorures dans lesquels les cations métalliques appartiennent aux groupes IA, IIA et IIB de la classification périodique des éléments. A titre d'exemples de cations convenant pour la mise en oeuvre du procédé de l'invention, on peut citer plus particulièrement parmi les cations du groupe IA, le lithium, le sodium, le potassium et le césium, parmi les cations du groupe IIA, le magnésium et le calcium, et parmi les cations du groupe IIB, le zinc. Parmi les sels précités, on choisit de préférence le fluorure de potassium et le fluorure de sodium. L'invention n'exclut pas la mise en oeuvre de sels doubles tels que les fluorures doubles d'aluminium et de sodium ou potassium et les fluosilicates de sodium ou potassium.

Lesdits fluorures d'onium, avantageusement employés comme sels apportant des anions fluorures dans le procédé selon l'invention, sont préférentiellement choisis parmi les fluorures d'ammonium, les fluorures de phosphonium, les fluorures d'imidazolium, les fluorures de guanidinium et les fluorures de pyridinium, pris seul ou en mélange.

Les fluorures d'ammonium et les fluorures de phosphonium sont des sels dont le cation répond en particulier à la formule (III) suivante : 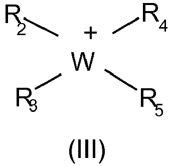 dans laquelle :
- W représente N ou P,
- R₂, R₃, R₄, et R₅, identiques ou différents représentent :
   - un groupe alkyle, linéaire ou ramifié, ayant 1 à 16 atomes de carbone et éventuellement substitué par un ou plusieurs hétéroatomes ou groupements phényle, hydroxyle, halogène, nitro, alkoxy ou alkoxycarbonyle, les groupements alkoxy ayant 1 à 4 atomes de carbone ;
   - un groupe alcényle, linéaire ou ramifié, ayant 2 à 12 atomes de carbone ;
   - un groupe aryle ayant 6 à 10 atomes de carbone, éventuellement substitué par un ou plusieurs hétéroatomes ou groupements alkyle ayant 1 à 4 atomes de carbone, alkoxy, alkoxycarbonyle, le groupe alkoxy ayant 1 à 4 atomes de carbone, ou halogène.
Les fluorures d'ammonium et les fluorures de phosphonium, préférentiellement employés pour la mise en oeuvre du procédé selon l'invention, présentent un cation qui répond à la formule (III) dans laquelle W est un atome d'azote ou de phosphore et R₂, R₃, R₄ et R₅, identiques ou différents, sont choisis parmi un groupe alkyle, linéaire ou ramifié, ayant 1 à 4 atomes de carbone et un groupe benzyle. A titre d'exemples plus spécifiques, on peut citer les fluorures de tétrabutylammonium, de méthyltri(n-butyl)ammonium, de N-méthyl-N,N,N-trioctylammonium, de triméthylphénylphosphonium, de tétrabutylphosphonium, de méthyltri(n-butyl)phosphonium, de méthyltri(isobutyl)phosphonium, de diisobutyl-n-octylméthylphosphonium. On choisit préférentiellement le fluorure de tétrabutylammonium (R₂ = R₃ = R₄ = R₅ = butyle et W = N) et le fluorure de tétrabutylphosphonium (R₂ = R₃ = R₄ = R₅ = butyle et W = P).

Les fluorures d'imidazolium et les fluorures de pyridinium sont des sels apportant des anions fluorure et dont le cation répond respectivement à la formule (IV) ou la formule (V) ci-dessous : 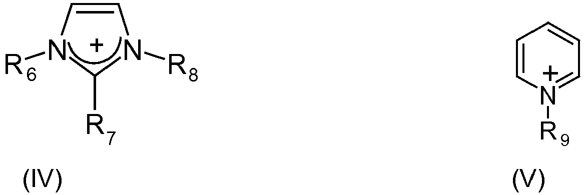 dans lesquelles :
- le groupe R₆ représente un groupe alkyle ayant de 1 à 20 atomes de carbone,
- le groupe R₇ représente un atome d'hydrogène, un groupe alkyle ayant de 1 à 4 atomes de carbone,
- le groupe R₈ représente un groupe alkyle ayant de 1 à 4 atomes de carbone,
- le groupe R₉ représente un groupe alkyle ayant de 1 à 6 atomes de carbone.
Parmi les cations répondant aux formules (IV) et (V), les cations 1-alkyl-2,3-diméthylimidazolium (R₆ = alkyle en C₁-C₂₀, R₇ = R₈ = méthyle), 1-alkyl-3-méthylimidazolium (R₆ = alkyle en C₁-C₂₀, R₇ = H, R₈ = méthyle) et 1-alkylpyridinium (R₉ = alkyle en C₁-C₆) sont préférés. Comme exemples plus spécifiques de fluorures d'imidazolium, on peut citer les fluorures de 1-alkyl-2,3-diméthylimidazolium tels que le fluorure de 1-éthyl-2,3-diméthylimidazolium, de 1-butyl-2,3-diméthylimidazolium, de 1-hexyl-2,3-diméthylimidazolium ; le tétrafluoroborate de 1-butyl-2,3-diméthylimidazolium, de 1-hexyl-2,3-diméthylimidazolium ; les fluorures de 1-alkyl-3-méthylimidazolium tels que le fluorure de 1-éthyl-3-méthylimidazolium, de 1-hexyl-3-méthylimidazolium, de 1-octyl-3-méthylimidazolium, de 1-décyl-3-méthylimidazolium, de 1-dodécyl-3-méthylimidazolium, de 1-tétradécyl-3-méthylimidazolium, de 1-hexadécyl-3-méthylimidazolium, de 1-octadécyl-3-méthylimidazolium ; l'hexafluorophosphate de 1-butyl-3-méthylimidazolium, de 1-hexyl-3-méthylimidazolium, de 1-octyl-3-méthylimidazolium ; le tétrafluoroborate de 1-butyl-3-méthylimidazolium, de 1-hexyl-3-méthylimidazolium. Les fluorures d'imidazolium préférés sont l'hexafluorophosphate de 1-butyl-3-méthylimidazolium, le tétrafluoroborate de 1-butyl-3-méthylimidazolium. Comme exemples plus spécifiques de fluorures de pyridinium, on peut citer les sels de 1-alkylpyridinium tels que le fluorure de 1-éthylpyridinium, de 1-butylpyridinium, de 1-hexylpyridinium ; l'hexafluorophosphate de 1-butylpyridinium, de 1-hexylpyridinium ; le tétrafluoroborate de 1-butylpyridinium, de 1-hexylpyridinium.

Ledit composé halogénosulfoxyde de formule (I0) ou ledit composé halogénosulfonyle de formule (II0) employé comme réactif pour la mise en oeuvre du procédé selon l'invention se présente sous forme acide ou sous forme salifiée. Sous forme salifiée, on emploie avantageusement des sels des métaux alcalins et alcalino-terreux ou encore des sels organiques sous forme de liquide ionique, en particulier les sels d'onium et plus particulièrement les sels d'ammonium (très préférentiellement un sel de tétrabutylammonium), de phosphonium, d'imidazolium, de pyridinium, de guanidinium.

Conformément au procédé de l'invention, on opère la réaction de fluoration entre ledit composé de formule (I0) ou ledit composé de formule (II0) et le sel apportant l'anion fluorure en présence d'eau. La quantité dudit sel dans l'eau est telle qu'elle représente de 1% poids, de préférence de 10% poids jusqu'à saturation de l'eau à la température de réaction. La réaction peut également être conduite en milieu hydro-organique, en particulier en utilisant un solvant organique polaire ou apolaire.
La réaction de fluoration est avantageusement mise en oeuvre à une température comprise entre 20 et 160°C, de préférence entre 60 et 120°C. Elle est avantageusement conduite sous pression atmosphérique. Elle est conduite pendant une durée préférentiellement comprise entre quelques minutes et 20 heures, très préférentiellement entre 0,5 et 10 heures.

Selon le procédé de l'invention, le rapport molaire entre le nombre de mole dudit composé halogénosulfoxyde ou dudit composé halogénosulfoyde et le nombre de mole de sel exprimé en anion fluorure est compris entre 1 et 20 et de préférence entre 3 et 10.

Conformément au procédé de l'invention, la réaction de fluoration est conduite en milieu monophasique ou biphasique liquide/liquide.

Le procédé de l'invention est simple à mettre en oeuvre. Les réactifs peuvent être introduits dans n'importe quel ordre selon différentes variantes mais certaines sont préférées. Un mode de réalisation préféré consiste à introduire ledit composé de formule (I0) ou (II0) sur un mélange formé de l'eau et dudit sel apportant au moins ledit anion fluorure. L'addition dudit composé de formule (I0) ou (II0) est préférentiellement effectuée une fois que ledit mélange aqueux a été chauffé à une température comprise dans la gamme précitée.

Le composé fluoré et soufré de formule (I) ou (II) obtenu à l'issue de la mise en oeuvre de ladite réaction de fluoration se présente sous forme de molécule neutre ou bien sous forme salifiée. La forme salifiée est obtenue lorsque ledit composé halogénosulfoxyde ou ledit composé halogénosulfonyle possède un hydrogène acide dont le pKa dans l'eau est inférieur à 7. De manière préférée, on obtient ledit composé fluoré et soufré de formule (I) ou (II) sous forme salifiée. Par exemple, dans la mise en oeuvre du mode de réalisation préféré du procédé selon l'invention consistant à employer l'acide bis-chlorosulfonylimide ou l'un de ses sels comme composé de formule (II0), on obtient à l'issue de la mise en oeuvre de ladite réaction de fluoration un sel de l'acide bis-fluorosulfonylimide, par exemple un sel de potassium (bis-fluorosulfonylimidure de potassium) lorsque ledit sel apportant ledit anion fluorure est le fluorure de potassium.

Le procédé de préparation dudit composé fluoré et soufré selon l'invention est avantageusement conduit dans un appareillage susceptible de résister à la corrosion du milieu réactionnel.
A cet effet, on choisit des matériaux pour la partie en contact avec le milieu réactionnel résistant à la corrosion comme les alliages à base de molybdène, chrome, cobalt, fer, cuivre manganèse, titane, zirconium, aluminium, carbone et tungstène vendus sous les marques HASTELLOY® ou les alliages de nickel, chrome, fer, manganèse additivés de cuivre et/ou molybdène commercialisés sous la dénomination INCONEL®, MONEL™ et plus particulièrement les alliages HASTELLOY C 276 ou INCONEL 600, 625 ou 718. On peut choisir également les aciers inoxydables, tels que les aciers austénitiques [Robert H. Perry et al, Perry's Chemical Engineers' Handbook, Sixth Edition (1984), page 23-44] et plus particulièrement les aciers inoxydables 304, 304 L, 316 ou 316 L. On met en oeuvre un acier ayant une teneur en nickel au plus de 22 % en masse, de préférence comprise entre 6 et 20 %, et plus préférentiellement comprise entre 8 et 14 %. Les aciers 304 et 304 L ont une teneur en nickel variant entre 8 et 12 % et les aciers 316 et 316 L ont une teneur en nickel variant entre 10 et 14 %. On choisit plus particulièrement les aciers 316 L.
On peut aussi faire appel à un appareillage constitué ou revêtu d'un composé polymérique résistant à la corrosion du milieu réactionnel. On peut citer notamment les matériaux tels que PTFE (polytétrafluoroéthylène ou Teflon) ou PFA (résines perfluoroalkyles). On ne sortira pas du cadre de l'invention à utiliser un matériau équivalent.
Comme autres matériaux susceptibles de convenir pour être en contact avec le milieu réactionnel, on peut également mentionner les dérivés du graphite.

Lorsque le composé fluoré et soufré de formule (I) ou (II) obtenu à l'issue de la mise en oeuvre de ladite réaction de fluoration est immiscible dans le milieu réactionnel, en particulier lorsqu'il s'agit d'une molécule neutre ou d'un sel dudit composé immiscible dans le milieu réactionnel, par exemple un sel d'onium, ledit composé est isolé, purifié, éventuellement transformé selon des techniques classiques connues de l'Homme du métier.

La présente invention a également pour objet un procédé de préparation d'au moins un sel dudit composé fluoré et soufré de formule (I) ou (II) à partir du procédé de préparation dudit composé de formule (I) ou (II) tel que décrit ci-dessus. Ledit sel dudit composé fluoré et soufré de formule (I) ou (II) est préférentiellement un sel d'un métal alcalin (Li, Na, K, Rb, Cs), un sel d'un métal alcalino-terreux, un sel d'un métal de transition ou un sel choisi parmi les lanthanides. De manière préférée, il s'agit d'un sel d'un métal alcalin, très préférentiellement d'un sel de lithium. En particulier, lorsque ledit composé fluoré et soufré de formule (I) ou (II) est un sel de l'acide bis-fluorosulfonylimide, on prépare avantageusement le bis-fluorosulfonylimidure de lithium (LiFSI). Le procédé de préparation d'au moins dudit sel dudit composé fluoré et soufré de formule (I) ou (II) selon l'invention comprend la préparation dudit composé de formule (I) ou (II) sous forme salifiée selon le procédé décrit ci-dessus suivie d'une étape d'extraction liquide/liquide puis de la mise en oeuvre soit d'un enchaînement comprenant les étapes d'acidification, de récupération de l'acide obtenu et de neutralisation soit d'une réaction d'échange de cations.

Le procédé de préparation d'au moins dudit sel dudit composé fluoré et soufré de formule (I) ou (II) selon l'invention est adapté lorsque ledit composé de formule (I) ou (II) obtenu à l'issue de ladite réaction de fluoration se trouve sous forme d'un sel miscible dans le milieu réactionnel aqueux. Ledit milieu réactionnel est soumis à une étape d'extraction liquide/liquide de manière à extraire ledit sel dudit composé de formule (I) ou (II) obtenu à l'issue de ladite réaction de fluoration. De manière plus précise, ledit milieu réactionnel est mis en contact avec un sel d'onium conduisant à la formation d'une phase organique comprenant le complexe résultant de la réaction dudit sel miscible dudit composé de formule (I) ou (II) obtenu à l'issue de ladite réaction de fluoration avec le sel d'onium et une phase aqueuse comprenant les différents sels en particulier celui résultant de la réaction du cation dudit sel miscible avec l'anion de l'onium.

Ledit sel d'onium avantageusement employé pour la mise en oeuvre de l'extraction liquide/liquide est préférentiellement choisi parmi les sels d'ammonium, les sels de phosphonium, les sels d'imidazolium, les sels de guanidinium et les sels de pyridinium, pris seul ou en mélange. Les formules de ces sels ont été précisées plus haut dans la présente description.

Ladite étape d'extraction liquide/liquide est mise en oeuvre en présence d'un solvant organique, préférentiellement un solvant organique halogéné, par exemple le dichlorométhane ou le dichloroéthane. Elle est conduite à une température comprise entre 10 et 100°C, préférentiellement entre 20 et 40°C.

Ladite étape d'extraction liquide/liquide a déjà été décrite dans la demande de brevet FR 2.933.693.

Ledit complexe obtenu est présent dans la phase organique et on procède à une séparation des phases aqueuse et organique, notamment par décantation, de manière à récupérer un liquide ionique dudit composé de formule (I) ou (II).

Selon un premier mode de réalisation du procédé de préparation dudit sel dudit composé fluoré et soufré de formule (I) ou (II) selon l'invention, on procède à l'acidification dudit complexe obtenu dans ladite phase organique de manière à en libérer la fonction acide. Ladite étape d'acidification est opérée au moyen d'un traitement à l'aide d'un acide de Brönstedt fort, par exemple l'acide sulfurique, l'acide chlorhydrique, l'acide phosphorique, l'acide sulfonique ou l'acide nitrique. La stoechiométrie en protons apportés par l'acide par rapport audit complexe varie de 1 à 10 équivalents molaires, de préférence de 1 à 4 équivalents molaires. L'acidification est préférentiellement conduite à une température comprise entre 10 et 50°C.
A l'issue de ladite étape d'acidification, ledit composé de formule (I) ou (II) se trouve sous forme acide dans la phase organique. Il est récupéré selon des techniques classiques connues de l'homme du métier, en particulier par distillation ou par extraction. Ledit composé de formule (I) ou (II) sous forme acide est très préférentiellement récupéré par distillation. Le solvant organique employé pour la mise en oeuvre de l'étape d'extraction liquide/liquide, préalable à l'étape de décantation, est d'abord distillé à pression atmosphérique, puis ledit composé de formule (I) ou (II) sous forme acide est distillé sous pression réduite. La mise en oeuvre de ladite étape de distillation est avantageuse car elle permet d'atteindre ledit composé avec la haute pureté requise pour les applications électroniques. Ledit composé de formule (I) ou (II) sous forme acide ainsi obtenu sous forme purifiée est mis en solution dans un solvant organique pour être soumis à une étape de neutralisation. Ledit solvant organique est préférentiellement un solvant aliphatique chloré ou un solvant aromatique. En particulier, il s'agit du dichlorométhane ou du dichloroéthane. La neutralisation est réalisée par traitement avec un hydroxyde métallique, un chlorure métallique ou un hydrure métallique, le cation métallique associé à l'anion hydroxyde étant un cation monovalent ou divalent, de préférence le cation lithium. De manière préférée, la neutralisation est réalisée en présence d'hydroxyde de lithium ou de chlorure de lithium. La neutralisation est conduite à une température comprise entre 10 et 100°C, de préférence entre 10 et 40°C. Ladite étape de neutralisation est aisée à mettre en oeuvre. De manière avantageuse, ledit composé de formule (I) ou (II) sous forme acide obtenu après distillation est mis en solution dans un solvant organique puis une solution aqueuse d'hydroxyde, de chlorure ou d'hydrure métallique est ajoutée. Les phases organique et aqueuse sont séparées par décantation ou filtration et la phase organique est évaporée. On récupère avantageusement le sel dudit composé fluoré et soufré de formule (I) ou (II) à partir de la phase aqueuse.
Selon un deuxième mode de réalisation du procédé de préparation dudit sel dudit composé fluoré et soufré de formule (I) ou (II) selon l'invention, on procède à une réaction d'échange de cations sur le complexe obtenu après ladite étape de décantation explicitée plus haut dans la présente description. Ladite réaction d'échange de cations est opérée par la mise en contact de la phase organique comprenant ledit complexe avec une solution aqueuse d'hydroxyde métallique ou d'halogénure métallique, le cation métallique associé à l'anion hydroxyde ou halogénure étant monovalent ou divalent. De manière préférée, ledit hydroxyde métallique est l'hydroxyde de lithium. La stoechiométrie en cation métallique par rapport audit complexe est comprise entre 0,5 et 5 équivalents molaires, de préférence entre 1 et 2. Ladite réaction d'échange est conduite à une température comprise entre 10 et 100°C, de préférence entre 10 et 40°C. En fin de réaction, les phases organique et aqueuse sont séparées par décantation. La phase organique est évaporée et ledit sel dudit composé fluoré et soufré de formule (I) ou (II) obtenu à partir de la phase aqueuse est préférentiellement séché sous vide.

On décrit ici également l'utilisation dudit composé fluoré et soufré de formule F-SO-R (I) ou F-SO₂-R (II) ou d'un sel dudit composé fluoré et soufré de formule (I) ou (II) préparés selon les procédés décrits ci-dessus comme sels d'électrolyte, comme précurseurs d'agent anti-statique ou encore comme précurseurs de tensio-actif. En particulier, ledit composé de formule (I) ou (II) ou ses sels sont avantageusement employés comme électrolytes pour la fabrication de batteries, dans le domaine de l'électrochromisme et de l'électronique. Ils sont avantageusement employés comme agents antistatiques pour la fabrication d'adhésifs sensibles à la pression (PSA : pressure sensitive adhesives). En tant qu'agent anti-statique, ils peuvent encore être employés comme composants de lubrifiants. Ils sont utilisés dans les matériaux optiques tels que les appareils électroluminescents et entrent dans la composition de panneaux photovoltaïques.

On donne ci-après des exemples de réalisation de l'invention. Ces exemples sont donnés à titre illustratif et sans caractère limitatif.

### Exemples 1 à 5 :

Dans un réacteur en verre, une solution de fluorure de potassium en solution aqueuse est portée à la température de réaction. Le bis-chlorosulfonylimide, noté CSIH, (10 grammes, 47 mmoles) est additionné en 30 secondes et l'agitation est maintenue pendant 2 heures à la température de réaction. Le milieu réactionnel est dilué dans l'eau pour l'analyse effectuée par RMN ¹⁹F afin de calculer le rendement en bis-fluorosulfonylimidure de potassium (FSIK).

Le tableau suivant rassemble les conditions opératoires utilisées et les résultats obtenus :

| Exemple n° | Stoechiométrie KF (équivalents) | Rapport molaire H2O/KF | Température (°C) | Conversion CSIH (%) | Rendement FSIK (%) |
|---|---|---|---|---|---|
| 1 | 10 | 2,8 | 100 | 100% | 38% |
| 2 | 10 | 2,7 | 75 | 100% | 51% |
| 3 | 10 | 2,3 | 50 | 100% | 65% |
| 4 | 15 | 2,4 | 50 | 100% | 60% |
| 5 | 20,8 | 2,5 | 50 | 100% | 65% |

### Exemple 6 (comparatif) : fluoration par le fluorure de zinc en milieu organique

Le fluorure de zinc (5,1g ; 49 mmoles) est mis en solution dans 90 grammes de valéronitrile. Le bis-chlorosulfonylimide est ensuite ajouté et l'agitation est maintenue 24 heures à température ambiante.

Une analyse RMN montre une conversion du bis-chlorosulfonylimide de 100%, et le rendement en sel de zinc du bis-fluorosulfonylimide est de 12%.

### Exemple 7 :

Dans un réacteur en verre, une solution de fluorure de potassium (27 g, 0,46 moles) en solution aqueuse (23 g d'eau) est portée à la température de 100°C. Le bis-chlorosulfonylimidure de potassium, noté CSIK, (11,8 grammes, 46 mmoles) est additionné en 30 secondes et l'agitation est maintenue pendant 2 heures à cette température de réaction. Le milieu réactionnel est dilué dans l'eau et ramené à température ambiante. L'analyse RMN ¹⁹F indique que le rendement obtenu en bis-fluorosulfonylimidure de potassium (FSIK) est de 69%.

### Exemple 8 :

A une solution de fluorure de potassium (27 g, 0,46 moles) dans l'eau (23 g) porté à la température de 120°C est ajouté le bis-chlorosulfonylimidure de *N*,*N*,*N*-tri-*n*-octyl-*N*-méthyl ammonium (27 g; 46 mmoles). Le milieu est laissé sous agitation pendant deux heures. Après retour à température ambiante, l'analyse RMN ¹⁹F indique que le rendement de bis-fluorosulfonylimidure *N,N,N-*tri-*n*-octyl-*N*-méthyl ammonium est de 49%.

### Exemple 9 :

A une solution de fluorure de potassium (27 g, 0,46 moles) dans l'eau (23 g) porté à la température de 120°C est ajouté le bis-chlorosulfonylimidure de potassium (10 g; 46 mmoles). Le milieu est laissé sous agitation pendant 30 minutes. Après retour à température ambiante, l'analyse RMN ¹⁹F indique que le rendement de bis-fluorosulfonylimidure de potassium est de 76%.

## Revendications

1. Procédé de préparation d'un composé fluoré et soufré de formule F-SO-R (I) ou F-SO₂-R (II) comprenant la réaction, en présence d'eau, d'au moins un sel apportant un anion fluorure et d'au moins un composé halogénosulfoxyde de formule X-SO-R' (I0) respectivement d'un composé halogénosulfonyle de formule X-SO₂-R' (II0), où X est un atome d'halogène différent du fluor et R et R' sont chacun un groupement lié par une liaison covalente à l'atome de soufre, ladite liaison liant ledit atome de soufre avec un atome d'azote, dans lequel le groupement R est choisi dans le groupe constitué par :
- un groupement -NHSO₂F,
- un groupement -NHSOF,
- un groupement - NM'SO₂F,
- un groupement - NM'SOF,
- un groupement -NR₁R₁₀,
- un groupement -NHSO₂R₁,
- un groupement -NHSOR₁,
- un groupement -NHCOR₁
où M' est un métal alcalin ou alcalino-terreux ou un ion onium, et R₁ et R₁₀, identiques ou différents, sont choisis dans le groupe constitué par un atome d'hydrogène, une chaîne hydrocarbonée linéaire, ramifiée ou cyclique, saturée ou insaturée ayant de 1 à 15 atomes de carbone, une chaîne fluoroalkyle, perfluoroalkyle ou fluoroalkényle ayant de 1 à 15 atomes de carbone et un groupement aromatique.

2. Procédé de préparation d'un composé fluoré et soufré de formule F-SO-R (I) ou F-SO₂-R (II) selon la revendication 1 dans lequel on utilise un composé halogénosulfonyle de formule (II0) X-SO₂-NH-SO₂-X de manière à préparer le composé F-SO₂-NH-SO₂-F (II).

3. Procédé de préparation d'un composé fluoré et soufré de formule F-SO-R (I) ou F-SO₂-R (II) selon la revendication 1 dans lequel on utilise un composé halogénosulfonyle de formule (II0) X-SO₂-NH-SO₂-CF₃ de manière à préparer le composé F-SO₂-NH-SO₂-CF₃ (II).

4. Procédé de préparation d'un composé fluoré et soufré de formule F-SO-R (I) ou F-SO₂-R (II) selon l'une des revendications 1 à 3 dans lequel ledit sel apportant un anion fluorure est choisi parmi les fluorures métalliques, les fluorures d'onium et leurs mélanges.

5. Procédé de préparation d'un composé fluoré et soufré de formule F-SO-R (I) ou F-SO₂-R (II) selon la revendication 3 dans lequel ledit sel apportant un anion fluorure est le fluorure de potassium.

6. Procédé de préparation d'un composé fluoré et soufré de formule F-SO-R (I) ou F-SO₂-R (II) selon l'une des revendications 1 à 5 dans lequel ledit composé halogénosulfoxyde de formule (I0) ou ledit composé halogénosulfonyle de formule (II0) se présente sous forme acide.

7. Procédé de préparation d'un composé fluoré et soufré de formule F-SO-R (I) ou F-SO₂-R (II) selon l'une des revendications 1 à 5 dans lequel ledit composé halogénosulfoxyde de formule (I0) ou ledit composé halogénosulfonyle de formule (II0) se présente sous forme salifiée, de préférence sous forme de sel de métal alcalin, de sel de métal alcalino-terreux ou de sel d'onium.

8. Procédé de préparation d'un composé fluoré et soufré de formule F-SO-R (I) ou F-SO₂-R (II) selon l'une des revendications 1 à 7 dans lequel on obtient ledit composé fluoré et soufré de formule F-SO-R (I) ou F-SO₂-R (II) sous forme salifiée, de préférence sous la forme d'un sel de potassium.

9. Procédé de préparation d'au moins un sel dudit composé fluoré et soufré de formule (I) ou (II) comprenant la préparation dudit composé de formule (I) ou (II) sous forme salifiée selon le procédé tel que défini dans les revendications 1 à 8 suivie d'une étape d'extraction liquide/liquide puis de la mise en oeuvre d'un enchaînement comprenant les étapes d'acidification, de récupération de l'acide obtenu et de neutralisation.

10. Procédé de préparation d'au moins un sel dudit composé fluoré et soufré de formule (I) ou (II) comprenant la préparation dudit composé de formule (I) ou (II) sous forme salifiée selon le procédé tel que défini dans les revendications 1 à 8 suivie d'une étape d'extraction liquide/liquide puis de la mise en oeuvre d'une réaction d'échange de cations.

11. Procédé de préparation d'au moins un sel dudit composé fluoré et soufré de formule (I) ou (II) selon la revendication 9 ou la revendication 10 dans lequel ledit sel dudit composé fluoré et soufré de formule (I) ou (II) est un sel d'un métal alcalin, un sel d'un métal alcalino-terreux, un sel d'un métal de transition ou un sel choisi parmi les lanthanides.

12. Procédé de préparation d'au moins un sel dudit composé fluoré et soufré de formule (I) ou (II) selon l'une des revendications 10 à 11 dans lequel on prépare le bis-fluorosulfonylimidure de lithium.

## Patentansprüche

1. Verfahren zur Herstellung einer Fluor- und Schwefelverbindung mit der Formel F-SO-R (I) oder F-SO₂-R (II), umfassend das Umsetzen, in Anwesenheit von Wasser, mindestens eines Salzes, das ein Fluoranion einbringt, und mindestens einer Halogensulfoxidverbindung mit der Formel X-SO-R' (I0) bzw. einer Halogensulfonylverbindung mit der Formel X-SO₂-R (110), wobei X ein von Fluor verschiedenes Halogenatom ist, und R und R' jeweils eine Gruppe sind, die durch eine kovalente Bindung an das Schwefelatom gebunden ist, wobei die Bindung das Schwefelatom an ein Stickstoffatom bindet, wobei die Gruppe R ausgewählt ist aus der Gruppe bestehend aus:
- einer Gruppe -NHSO₂F,
- einer Gruppe -NHSOF,
- einer Gruppe -NM'SO₂F,
- einer Gruppe -NM'SOF,
- einer Gruppe -NR₁R₁₀,
- einer Gruppe -NHSO₂R₁,
- einer Gruppe -NHSOR₁,
- einer Gruppe -NHCOR₁,
wobei M' ein Alkalimetall oder Erdalkalimetall oder ein Oniumion ist, und R₁ oder R₁₀, identisch oder verschieden, ausgewählt sind aus der Gruppe bestehend aus einem Wasserstoffatom, einer linearen, verzweigten oder cyclischen, gesättigten oder ungesättigten Kohlenwasserstoffkette mit 1 bis 15 Kohlenstoffatomen, einer Fluoralkyl-Perfluoralkyl- oder Fluoralkenylkette mit 1 bis 15 Kohlenstoffatomen und einer aromatischen Gruppe.

2. Verfahren zur Herstellung einer Fluor- und Schwefelverbindung mit der Formel F-SO-R (I) oder F-SO₂-R (II) nach Anspruch 1,
wobei eine Halogensulfonylverbindung mit der Formel (II0) X-SO₂-NH-SO₂-X verwendet wird, um die Verbindung F-SO₂-NH-SO₂-F (II) herzustellen.

3. Verfahren zur Herstellung einer Fluor- und Schwefelverbindung mit der Formel F-SO-R (I) oder F-SO₂-R (II) nach Anspruch 1,
wobei eine Halogensulfonylverbindung mit der Formel (II0) X-SO₂-NH-SO₂-CF₃ verwendet wird, um die Verbindung F-SO₂-NH-SO₂-CF₃ (II) herzustellen.

4. Verfahren zur Herstellung einer Fluor- und Schwefelverbindung mit der Formel F-SO-R (I) oder F-SO₂-R (II) nach einem der Ansprüche 1 bis 3,
wobei das Salz, das ein Fluoranion einbringt, aus Metallfloriden, Oniumfluoriden und ihren Mischungen ausgewählt wird.

5. Verfahren zur Herstellung einer Fluor- und Schwefelverbindung mit der Formel F-SO-R (I) oder F-SO₂-R (II) nach Anspruch 3,
wobei das Salz, das ein Fluoranion einbringt, Kaliumfluorid ist.

6. Verfahren zur Herstellung einer Fluor- und Schwefelverbindung mit der Formel F-SO-R (I) oder F-SO₂-R (II) nach einem der Ansprüche 1 bis 5,
wobei die Halogensulfoxidverbindung mit der Formel (I0) oder die Halogensulfonylverbindung mit der Formel (II0) in der Form einer Säure vorhanden ist.

7. Verfahren zur Herstellung einer Fluor- und Schwefelverbindung mit der Formel F-SO-R (I) oder F-SO₂-R (II) nach einem der Ansprüche 1 bis 5,
wobei die Halogensulfoxidverbindung mit der Formel (I0) oder die Halogensulfonylverbindung mit der Formel (II0) in versalzter Form, vorzugsweise in der Form eines Alkalimetallsalzes, eines Erdalkalimetallsalzes oder Oniumsalzes, vorhanden ist.

8. Verfahren zur Herstellung einer Fluor- und Schwefelverbindung mit der Formel F-SO-R (I) oder F-SO₂-R (II) nach einem der Ansprüche 1 bis 7,
wobei die Fluor- und Schwefelverbindung mit der Formel F-SO-R (I) oder F-SO₂-R (II) in versalzter Form, vorzugsweise in der Form eines Kaliumsalzes, erhalten werden.

9. Verfahren zur Herstellung mindestens eines Salzes einer Fluor- und Schwefelverbindung mit der Formel (I) oder (II), umfassend die Herstellung der Verbindung mit der Formel (I) oder (II) in versalzter Form gemäß einem Verfahren, wie in den Ansprüchen 1 bis 8 definiert, gefolgt von einem Flüssig/ Flüssig-Extraktionsschritt, dann von der Durchführung einer Verkettung, umfassend die Schritte einer Ansäuerung, einer Gewinnung der erhaltenen Säure und einer Neutralisation.

10. Verfahren zur Herstellung mindestens eines Salzes der Fluor- und Schwefelverbindung mit der Formel (I) oder (II), umfassend die Herstellung der Verbindung mit der Formel (I) oder (II) in versalzter Form gemäß dem Verfahren, wie in den Ansprüchen 1 bis 8 definiert, gefolgt von einem Flüssig/ Flüssig-Extraktionsschritt, dann von der Durchführung einer Kationenaustauschreaktion.

11. Verfahren zur Herstellung mindestens eines Salzes der Fluor- und Schwefelverbindung mit der Formel (I) oder (II) nach Anspruch 9 oder Anspruch 10,
wobei das Salz der Fluor- und Schwefelverbindung mit der Formel (I) oder (II) ein Alkalimetallsalz, ein Erdalkalimetallsalz, ein Übergangsmetallsalz oder ein aus Lanthaniden ausgewähltes Salz ist.

12. Verfahren zur Herstellung mindestens eines Salzes der Fluor- und Schwefelverbindung mit der Formel (I) oder (II) nach einem der Ansprüche 10 bis 11,
wobei Lithium-bisfluorsulfonylimid hergestellt wird.

## Claims

1. Process for preparing a fluorine-containing and sulfur-containing compound of formula F-SO-R (I) or F-SO₂-R (II) comprising the reaction, in the presence of water, of at least one salt providing a fluoride anion and of at least one halosulfoxide compound of formula X-SO-R' (10), respectively of a halosulfonyl compound of formula X-SO₂-R' (110), where X is a halogen atom other than fluorine and R and R' are each a group bonded via a covalent bond to the sulfur atom, said bond bonding said sulfur atom with a nitrogen atom, wherein the R group is selected from the group consisting of:
- an -NHSO₂F group,
- an -NHSOF group,
- an -NM'SO₂F group,
- an -NM'SOF group,
- an -NR₁R₁₀ group,
- an -NHSO₂R₁ group,
- an -NHSOR₁ group,
- an -NHCOR₁ group;
where M' is an alkali or alkaline-earth metal, and R₁ and R₁₀, which may be identical or different, are selected from the group consisting of a hydrogen atom, a saturated or unsaturated, linear, branched or cyclic hydrocarbon-based chain having from 1 to 15 carbon atoms, a fluoroalkyl, perfluoroalkyl or fluoroalkenyl chain having from 1 to 15 carbon atoms, and an aromatic group.

2. Process for preparing a fluorine-containing and sulfur-containing compound of formula F-SO-R (I) or F-SO₂-R (II) according to Claim 1, wherein a halosulfonyl compound of formula (110) X-SO₂-NH-SO₂-X is used so as to prepare the compound F-SO₂-NH-SO₂-F (II).

3. Process for preparing a fluorine-containing and sulfur-containing compound of formula F-SO-R (I) or F-SO₂-R (II) according to Claim 1, wherein a halosulfonyl compound of formula (110) X-SO₂-NH-SO₂-CF₃ is used so as to prepare the compound F-SO₂-NH-SO₂-CF₃ (II).

4. Process for preparing a fluorine-containing and sulfur-containing compound of formula F-SO-R (I) or F-SO₂-R (II) according to one of Claims 1 to 3, wherein said salt providing a fluoride anion is selected from metal fluorides, onium fluorides and mixtures thereof.

5. Process for preparing a fluorine-containing and sulfur-containing compound of formula F-SO-R (I) or F-SO₂-R (II) according to Claim 3, wherein said salt providing a fluoride anion is potassium fluoride.

6. Process for preparing a fluorine-containing and sulfur-containing compound of formula F-SO-R (I) or F-SO₂-R (II) according to one of Claims 1 to 5, wherein said halosulfoxide compound of formula (10) or said halosulfonyl compound of formula (II0) is in acid form.

7. Process for preparing a fluorine-containing and sulfur-containing compound of formula F-SO-R (I) or F-SO₂-R (II) according to one of Claims 1 to 5, wherein said halosulfoxide compound of formula (10) or said halosulfonyl compound of formula (II0) is in salified form, preferably in alkali metal salt, alkaline-earth metal salt or onium salt form.

8. Process for preparing a fluorine-containing and sulfur-containing compound of formula F-SO-R (I) or F-SO₂-R (II) according to one of Claims 1 to 7, wherein said fluorine-containing and sulfur-containing compound of formula F-SO-R (I) or F-SO₂-R (II) is obtained in salified form, preferably in the form of a potassium salt.

9. Process for preparing at least one salt of said fluorine-containing and sulfur-containing compound of formula (I) or (II), comprising the preparation of said compound of formula (I) or (II) in salified form according to the process as defined in Claims 1 to 8, followed by a step of liquid/liquid extraction, and then the implementation of a sequence comprising the steps of acidification, of recovery of the acid obtained and of neutralization.

10. Process for preparing at least one salt of said fluorine-containing and sulfur-containing compound of formula (I) or (II), comprising the preparation of said compound of formula (I) or (II) in salified form according to the process as defined in Claims 1 to 8, followed by a step of liquid/liquid extraction, and then the implementation of a cation exchange reaction.

11. Process for preparing at least one salt of said fluorine-containing and sulfur-containing compound of formula (I) or (II) according to Claim 9 or Claim 10, wherein said salt of said fluorine-containing and sulfur-containing compound of formula (I) or (II) is a salt of an alkali metal, a salt of an alkaline-earth metal, a salt of a transition metal or a salt selected from lanthanides.

12. Process for preparing at least one salt of said fluorine-containing and sulfur-containing compound of formula (I) or (II) according to either of Claims 10 and 11, wherein lithium bis(fluorosulfonyl)imide is prepared.
